(19) Europäisches Patentamt
European Patent Office
Office européen des brevets

(11) Publication number: 0 186 365
A2

(12) EUROPEAN PATENT APPLICATION

(21) Application number: 85308942.3

(22) Date of filing: 09.12.85

(51) Int. Cl.[4]: C 12 P 7/02
C 12 P 7/24
//C12N9/04

(30) Priority: 12.12.84 GB 8431365

(43) Date of publication of application:
02.07.86 Bulletin 86/27

(84) Designated Contracting States:
DE FR IT NL

(71) Applicant: UNITED KINGDOM ATOMIC ENERGY AUTHORITY
11 Charles II Street
London SW1Y 4QP(GB)

(72) Inventor: Chadwick, Andrew Thomas
Pentrose Fieldside
Upton Didcot Oxon(GB)

(72) Inventor: Lambert, Nigel
15 Courtney Close
Chapel Brake Norwich(GB)

(72) Inventor: Pugh, Shirley Yvonne Ruth
30 Mallard Way Grove
Wantage Oxon(GB)

(72) Inventor: Thomson, Alan Russell
69 Bath Street
Abingdon Oxon(GB)

(74) Representative: Mansell, Keith Rodney
Patents Branch United Kingdom Atomic Energy
Authority 11 Charles II Street
London SW1Y 4QP(GB)

(54) **Preparation of alcohols.**

(57) Alcohols, such as aromatic and aliphatic alcohols, are prepared by reducing the corresponding aldehyde with an alcohol dehydrogenase enzyme and with a cofactor for the enzyme. The enzyme is immobilised by covalent bonding to a support material to ameliorate the problem of its inhibition by the aldehyde and hence increase its active life. The enzyme is preferably an enzyme of Enzyme Commission class 1.1.1.1. such as yeast alcohol dehydrogenase (YADH) or horse liver alcohol dehydrogenase (HLADH). The support material may, for example, be agarose or an oxirane-acrylic polymer.



Croydon Printing Company Ltd

0186365

# Preparation of Alcohols

This invention relates to a process for the preparation of alcohols by enzyme-catalysed reduction of aldehydes.

The enzyme yeast alcohol dehydrogenase which belongs to Enzyme Commission (EC) class 1.1.1.1., hereinafter referred to as "YADH" for brevity, is known to catalyse the oxidation of alcohols (alkanols) to aldehydes (alkanals) and the reduction of simple aldehydes to alcohols. It requires NAD/NADH cofactor. Thus the reduction proceeds according to the equation

$$RCHO + NADH + H^{+} \longrightarrow NAD^{+} + RCH_2OH$$

where R represents the residue of the simple aldehyde, for example acetaldehyde.

YADH is available commercially in various forms. See, for example, the February 1984 catalogue of Sigma Chemical Co. Ltd. which lists two crystallised, lyophilised forms and two immobilised (insolubilised) forms in which the enzyme is attached to agarose beads or to polyacrylamide, and also the February 1985 catalogue of Sigma Chemical Co Ltd.

The reduction is not of great interest for the preparation of simple aliphatic alcohols, since cheaper chemical methods are available. Thus, its use in relation to simple aldehydes has been largely for the purposes of assay for the cofactor or for the aldehyde.

This invention is concerned with the production of alcohols by the above-mentioned enzymatic reduction, preferably the production of aromatic alcohols such as cinnamyl alcohol, a product used widely in the perfume

industry. It is known that cinnamyl alcohol can be oxidised to cinnamaldehyde using YADH, see K. Ebisuzaki and E.S.G. Barron, Arch. Biochem. Biophys. 69, 555 (1957), but the reduction using YADH is not described therein.

The enzymatic reduction of aldehydes may, however, present a serious problem due to the aldehyde, whilst being a substrate for the enzyme, being a potent irreversible inhibitor. For example, when a solution of the enzyme was incubated with 5 mM cinnamaldehyde, 50% inhibition resulted in 12 minutes and total inhibition in about 30 minutes. (By contrast cinnamyl alcohol had no inhibitory effect even at 8 mM). Irreversible inhibition means that the enzyme is inhibited permanently by the substrate and loses its catalytic activity. Also, irreversible inhibition of YADH by other aldehydes, e.g. benzaldehyde, o, m and p tolualdehydes and salicylaldehyde, has been observed.

It has now been found, surprisingly, that if an alcohol dehydrogenase "(ADH)", e.g. of EC class 1.1.1.1., is immobilised by covalent bonding to a support material it may not readily undergo irreversible inhibition by aldehydes. This finding may make possible the reduction of aldehydes by such an enzyme on a preparative scale, and, particularly, the production of alcohols such as cinnamyl alcohol for the speciality chemicals market. Thus, the invention provides a process for the preparation of an alcohol which comprises reducing an aldehyde with an ADH effective to catalyse the reduction, immobilised by covalent bonding to a support material, and with a cofactor for the ADH.

The process of the invention may be applicable to an aldehyde which irreversibly inhibits the ADH in free solution, especially to aromatic and aliphatic aldehydes. Cinnamaldehyde is normally available in the trans form or

as a mixture of cis and trans forms in which the trans isomer predominates and the invention is applicable to the reduction of cinnamaldehyde in these forms.

The enzyme is preferably of Enzyme Commission class 1.1.1.1. whether it is derived from yeast (i.e. is a YADH) from horse liver (HLADH), or from another source. The enzyme may, however, be of Enzyme Commission Class 1.1.1.2 such as from Thermoanaerobium brockii, a thermostable organism.

It should be noted that different enzymes have, in most cases, different specificities in the practice of the invention. For example, YADH has been found to be suitable, as indicated above, for the reduction of trans-cinnamaldehyde, and HLADH has been found to be suitable for the reduction of a range of aldehydes as will be illustrated in the examples herein.

The support material and method of immobilisation can be any known for the particular enzyme in connection with the oxidation of ethanol to acetaldehyde. A preferred support material is agarose, which can be covalently bonded to the enzyme by the known cyanogen bromide method. Another preferred support material is "Eupergit C", which consists of "oxirane-acrylic beads" obtained by copolymerisation of glycidyl methacrylate and/or allyl glycidyl ether, methacrylamide and methylene-bisacrylamide. This support material is known in the art ex Rohm Pharma GmbH or, in the UK, ex Dumas Chemicals Ltd, Pantiles House, Nevill Street, Tunbridge Wells, Kent. These beads are reactable with the enzyme by adding a solution of the enzyme thereto at room temperature, and allowing the mixture to stand for 24 hours. The process is conveniently carried out in a column packed with the beads of support material carrying the immobilised enzyme thereon.

The reduction of the aldehyde can be carried out under any conditions appropriate to use of the ADH enzyme. The preferred pH range is 6 to 8, a pH of about 7.5 usually being most suitable.

An example of a cofactor for the ADH in the practice of the invention is NADH. Since the cofactor is oxidised during reduction of the aldehyde, it is preferable to arrange for recycling of the $NAD^+$ produced by an appropriate redox couple which will re-convert it to its reduced form. Examples of redox reactants providing such a couple are glucose-6-phosphate with glucose-6-phosphate dehydrogenase, formate anion with formate dehydrogenase, or the wholly chemical reductant sodium dithionite. Where an enzymatic couple is used, the enzyme is conveniently co-immobilised on the support material for the ADH.

The alcohol can be recovered by extraction into an appropriate solvent, e.g. dichloromethane in the case of cinnamyl alcohol, and evaporation of the solvent.

The following Examples illustrate the invention, where Examples 1, 2 and 4 relate to enzyme activity and Examples 3 and 5 relate to enzyme inhibition.

EXAMPLE 1

Baker's yeast YADH enzyme A 7011 from Sigma Chemical Co. Ltd. (100 mg) and glucose-6-phosphate dehydrogenase ("G6PDH") (3 mg) were co-immobilised on "Sepharose 4B" (Registered Trade Mark) beads from Pharmacia (6 ml) which are agarose beads activated with cyanogen bromide.

The beads and enzymes, in a buffer of 0.1M phosphate containing 0.5M potassium chloride, pH 7.5, (50 ml) were

rolled for 24 hours at 4°C. Approximately 60% of each enzyme was thus immobilised.

A substrate solution was prepared consisting of cinnamaldehyde (5mM) and NADH (1mM) in the same buffer (100 mM). The column was initially equilibrated with cinnamaldehyde, and glucose-6-phosphate (10mM) was added to the substrate solution to initiate the reaction. The substrate solution was continually pumped from a 20 ml reservoir thereof through a packed bed column of the immobilised YADH-G6PDH beads (4 ml) at a flow rate of 15 ml/min.

Progress of the reaction was monitored spectrometrically at 380 nm for NADH and by HPLC for the production of the cinnamyl alcohol.

After a start-up period of about 5 minutes, the NADH concentration was maintained constant at about 1mM and aldehyde was reduced quantitatively to alcohol over the first 30 minutes of the reaction.

The experiment was run for 5 days and 4 nights, but overnight, cinnamaldehyde alone was circulated through the column. This was found to bind to the column to some extent. Nevertheless, by elevating the G6P concentration to 20mM at the end of this period, the conversion of aldehyde to alcohol was still as high as 90%.

In comparative tests, it was determined that when YADH immobilised as described above (but without G6PDH) on "Sepharose 4B" beads and YADH in solution were incubated with cinnamaldehyde 5mM alone, the half-life of inactivation was 12 minutes for YADH in solution and 240 minutes for immobilised YADH.

EXAMPLE 2

Immobilised YADH-G6PDH beads were prepared as described in Example 1, substituting "Eupergit C" (3g) for Sepharose B beads, and using 2 mg of G6PDH. The "Eupergit C" beads required no preliminary activation. After 24 hours there resulted 23% immobilisation of G6PDH and 33% immobilisation of YADH.

A packed bed column was set up as in Example 1 except that 10 ml of beads were used and a flow rate of 2 ml/min was used. A 70% conversion of cinnamaldehyde to cinnamyl alcohol was obtained after one pass of the aldehyde through the column. (It should be noted that the comparable percentage conversion when using "Sepharose 4B" beads at a flow rate of 2 ml/min was about 95%).

EXAMPLE 3

(i) Immobilised Enzyme

YADH (15 mg; 200U/mg) was reacted with swelled CNBr-activated Sepharose 4B (Registered Trade Mark) beads (0.4 g; ex Pharmacia) and coupling carried out in sodium phosphate buffer (0.1M; pH 7.0) containing KCl (0.5 M) for 18 hours at 4°C. Approximately 90% of the enzyme was thus immobilised.

In order to test the inhibition of the immobilised enzyme by aldehyde, the beads (50 - 60 μl) were incubated with trans-cinnamaldehyde (1.3 ml; 10 mM) and a coupling buffer (3.95 ml; pH 7.0). This gave a final aldehyde concentration of 2 mM and a protein concentration of 0.16 mg/ml. The reaction mixture was incubated at 22 (±2)°C for 60 min with continuous gentle agitation. The immobilised enzyme was washed three times in coupling buffer (10 ml) at 22 (±2)°C and resuspended in eight times of its original

volume in coupling buffer. Enzyme activity was assayed immediately after washing using trans-cinnamaldehyde (5 mM) as assay substrate with a protein concentration of 0.2 mg/ml.

(ii) Soluble Enzyme

By way of comparison, stock enzyme solution (800 μl; 0.2 mg protein/ml) was incubated with cinnamaldehyde (200 μl; 10 mM). This also gave a final aldehyde concentration of 2 mM and a protein concentration of 0.16 mg/ml. The reaction mixture was incubated at 22 ($\pm$2)°C for 60 min with continuous gentle agitation. After incubation, the soluble enzyme was dialysed against coupling buffer (30 ml; 0.1M) at 4°C for 2½ hours with one change of buffer. Enzyme activity was assayed immediately after dialysis using trans-cinnamaldehyde (5 mM) as assay substrate with a protein concentration of 0.2 mg/ml.

A comparison of the results of parts (i) and (ii) of the example showed that the soluble enzyme was inhibited by 90% at a given protein concentration whereas the immobilised enzyme was only 50% inhibited at the same protein concentration.

EXAMPLE 4

Horse liver alcohol dehydrogenase or HLADH (50 mg; 1 U/mg) was reacted with swelled CNBr-activated Sepharose 4B (Registered Trade Mark) beads (0.8 g). Approximately 90% of the enzyme was thus immobilised.

The activity of the immobilised enzyme in the reduction of aldehydes to alcohols was determined in coupling buffer (0.1 M; pH 7.0) at 22 ($\pm$2)°C as the initial rate of decrease in absorbance at 380 nm over a 2 minute

time period using NADH (1mM) and aldehyde (2mM). The immobilised enzyme was found to be active in the reduction of the following aldehydes where activity in micromoles NADH/min/mg protein is given in parentheses: citronellal (0.75); p-anisaldehyde (1.55); piperonaldehyde (1.47); p-cuminaldehyde (0.94); citral (0.91); trans-cinnamaldehyde (1.36); p-hydroxybenzaldehyde (0.94); and salicylaldehyde (0.57).

EXAMPLE 5

The inhibition of the immobilised enzyme (prepared as in Example 4) by aldehydes was tested and compared with the soluble enzyme as described in Example 3. The results were as follows:

(a) assaying using trans-cinnamaldehyde following pre-incubation using a different aldehyde

Incubation of soluble HLADH with citronellal, p-cuminaldehyde, trans-cinnamaldehyde, p-anisaldehyde, citral and salicylaldehyde all resulted in reduced activity of the enzyme towards trans-cinnamaldehyde compared with controls lacking the aldehydes. In contrast, the immobilised enzyme gave total protection against inhibition by all of the above aldehydes;

(b) assaying using the aldehyde used in pre-incubation

The aldehydes listed in (a) above all exhibited greater inhibition than in (a) when using soluble HLADH. Except for citronellal, where 11% inhibition was recorded, immobilised HLADH was totally protected against inhibition by all of the aldehydes listed in (a).

Claims

1. A process for the preparation of an alcohol which comprises reducing an aldehyde with an alcohol dehydrogenase enzyme and with a cofactor for the enzyme, characterised in that the enzyme is immobilised by covalent bonding to a support material.

2. A process according to claim 1 wherein the enzyme is of Enzyme Commission class 1.1.1.1.

3. A process according to claim 2 wherein the enzyme is derived from yeast.

4. A process according to claim 2 wherein the enzyme is derived from equine liver.

5. A process according to any of the preceding claims wherein the support material is agarose or an oxirane-acrylic polmer.

6. A process according to any of the preceding claims wherein the alcohol is an aromatic alcohol.